# EUROPEAN PATENT SPECIFICATION

(11) **EP 3 662 962 B1**
(45) Date of publication and mention of the grant of the patent: **07.06.2023**
(21) Application number: 20152833.8
(22) Date of filing: 08.03.2013
(51) Int. Cl.: A61M 25/10

(54) **MEDICAL BALLOON INCLUDING A RADIOPAQUE WIRE FOR PRECISELY IDENTIFYING A WORKING SURFACE LOCATION**
MEDIZINISCHER BALLON MIT EINEM RÖNTGENDICHTEN DRAHT ZUR GENAUEN IDENTIFIZIERUNG EINER ARBEITSFLÄCHENPOSITION
BALLONNET MÉDICAL COMPRENANT UN FIL RADIO-OPAQUE POUR IDENTIFIER AVEC PRÉCISION L'EMPLACEMENT D'UNE SURFACE DE TRAVAIL

(30) Priority: 09.03.2012 NL 2008447; 09.03.2012 US 201261608897 P
(43) Date of publication of application: 10.06.2020
(62) Divisional of application: 18169029.8
(73) Proprietor: Clearstream Technologies Limited, Enniscorthy, County Wexford (IE)
(72) Inventor: Wall, Sean, Enniscorthy, County Wexford (IE)
(74) Representative: Hoffmann Eitle

(56) References cited:
- EP-A1- 0 850 659
- WO-A1-2005/096797
- WO-A1-2011/112863
- WO-A2-2007/132447
- WO-A2-2012/009486
- JP-A- 2008 067 994
- US-A- 5 085 636
- US-A1- 2002 198 559
- US-A1- 2005 255 317
- US-A1- 2006 224 114

## Description

### Technical Field

This disclosure relates generally to balloons for performing medical procedures, such as angioplasty and, more particularly, to a medical balloon having a predetermined portion, such as a working surface, that may be precisely located or identified during use.

### Background of the Invention

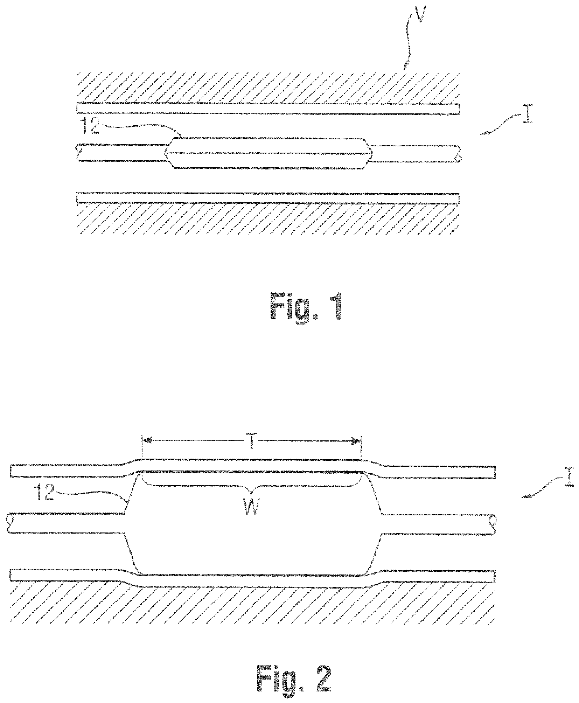

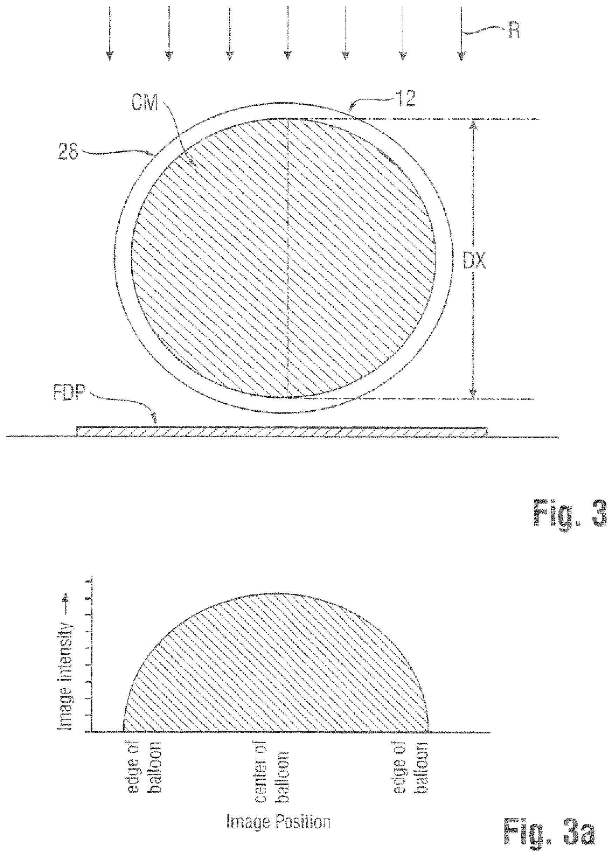

Balloons are routinely used to resolve or address flow restrictions or perhaps even complete blockages in tubular areas of the body, such as arteries or veins. In many clinical situations, the restrictions are caused by hard solids, such as calcified plaque, and require the use of high pressures to compact such blockages. Commercially available balloons employ complex technology to achieve high pressure requirements without sacrificing the profile of the balloon. Besides high pressure requirements, the balloons should also be resistant to puncture, easy to track and push, and present a low profile, especially when used for angioplasty.
In clinical practice, angioplasty balloons are expanded from a deflated, folded state to an expanded state within a vessel to treat a target area, such as a portion of the circumferential inner wall I of a blood vessel V, as shown in Figures 1 and 2. The inflation is traditionally completed using an X-ray contrast agent to provide better visibility under X-ray or other form of radiography during the interventional procedure, as illustrated in Figures 3 and 3a. Typically, a 70/30 percent mixture of contrast agent and saline is used to inflate the balloon during an angioplasty procedure. The arrows R in Fig. 3 indicate the direction of the X-ray beams. Fig. 3 further shows a conventional balloon 12 inflated with fluid containing contrast media. Further, reference sign CM refers to X-ray absorbing contrast media in the inflation fluid. Moreover, a fluoroscope detector plate FDP is shown. The length DX defines the long distance that X-ray beams travel through contrast media at the center of the balloon image. Fig. 3a shows the image intensity as a function of the image position.

In general, a desirable goal is to reduce inflation and deflation times required for balloons without sacrificing the profile of the balloons, especially for large volume balloons (which can require up to two minutes of inflation/deflation times with the contrast agent). Because of its relatively high viscosity, it would also be desirable to eliminate, or at least reduce the amount of, the contrast agent used in inflation/deflation of the balloons. The use of contrast agent prolongs the inflation/deflation times and also poses the iodine exposure to patients sensitive to iodine. In this regard, a non-radiopaque substance could be used in lieu of the contrast agent, such as for example saline or carbon dioxide, but such substances are invisible during X-ray imaging, and thus do not enhance visibility.

Furthermore, the physician performing the angioplasty procedure should be able to locate the position of the uninflated balloon with accuracy, so that the balloon will be properly positioned once inflated. This is conventionally accomplished by attaching marker bands on the catheter shaft in the region corresponding to the balloon working surface. This "working surface" is the surface along the portion of the balloon that is used to achieve the desired treatment effect, such as contacting the calcified plaque (which surface in the case of a balloon having conical or tapering sections at the proximal and distal ends is typically co-extensive with a generally cylindrical barrel section).

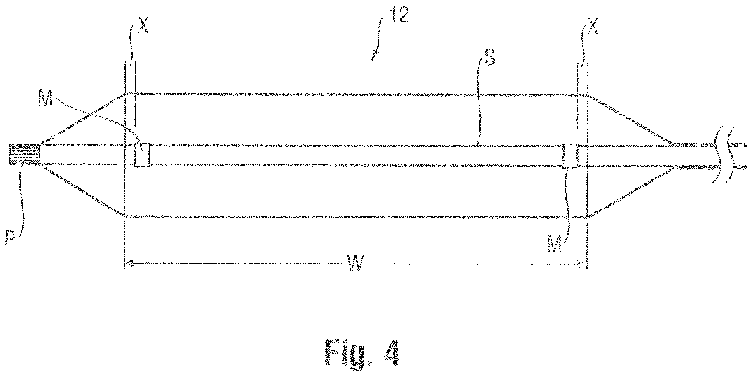

Misalignment of the marker bands during placement along the shaft sometimes results in their failure to correspond precisely to the extent of the working surface, as is shown in Figure 4 (note misalignment amount X between each interior marker band M carried by shaft S and working surface W of balloon 12, which also typically includes a radiopaque tip P at the distal end). Even upon exercising great care to position the markers properly on the underlying shaft in alignment with anticipated boundaries of the working surface when the balloon is inflated, there remains a tendency for mismatch due to several possible factors. One such factor may be the tolerance stack-ups arising as a consequence of the affixation of the balloon to the distal end of the catheter shaft. The balloon also has a tendency to grow in the longitudinal direction when inflated, especially with large and particularly long balloons. Another factor is the tendency of the portion of the catheter shaft within the balloon to bend or flex during inflation. This may lead to misalignment between radiopaque markers fixed to the shaft and the working surface.

Whatever the cause, the resulting misalignment may prevent the clinician from accurately identifying the location of the working surface of the balloon during an interventional procedure. This may lead to a geographic misplacement, or "miss," of the intended contact between the target area T and the working surface W of the balloon 12 (see Figure 2). It is especially desirable to avoid such an outcome when the balloon is designed to deliver a payload (such as a drug, stent, or both) or a working element to a specified location within the vasculature, since a miss may prolong the procedure (such as, for example, by requiring redeployment of the balloon 12 or the use of another balloon catheter in the case of a drug coated balloon).

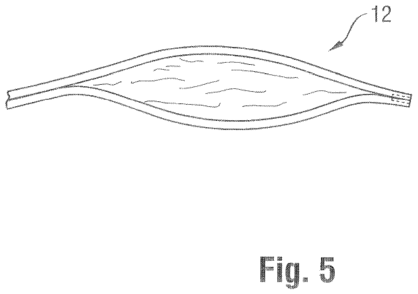

Upon deflation, the balloon may also be subject to a phenomenon known as "pancaking." In this condition, the balloon 12 folds down upon itself to a flattened state, as shown in Figure 5. This situation may cause the balloon to be viewed through fluoroscopy as perhaps still being in the inflated condition, since the full width of the balloon may still be perceived. This can give the clinician the false perception that the balloon remains inflated, when in fact it is not.

Accordingly, the need is identified for a balloon for which the working surface may be identified during an interventional procedure with enhanced precision. The solution would take into account the possible mismatch between fixed locations on the catheter shaft and the balloon to define the working surface, and would operate independent of the position of the portion of the catheter shaft within the balloon. The improved identification may also allow for the better detection of the false perception of deflation caused by pancaking. Overall, procedural efficiency would be enhanced without remarkably increasing cost or complexity, and in a manner that can be applied to many existing catheter technologies without extensive modification.

WO2012/009486 A2 discloses an inflatable medical device for transcutaneous heart valve implantation, such as balloons used for transcatheter aortic-valve implantation. The balloons can have high strength, fiber-reinforced walls.

### Summary of the Invention

The present invention is directed to the balloon catheter of claim 1. The dependent claims refer to preferred embodiments. An object of the disclosure is to provide a balloon for which the working surface may be identified during an interventional procedure with enhanced precision.

### Modes for Carrying Out the Invention

The description provided below and in regard to the figures applies to all embodiments unless noted otherwise, and features common to each embodiment are similarly shown and numbered.

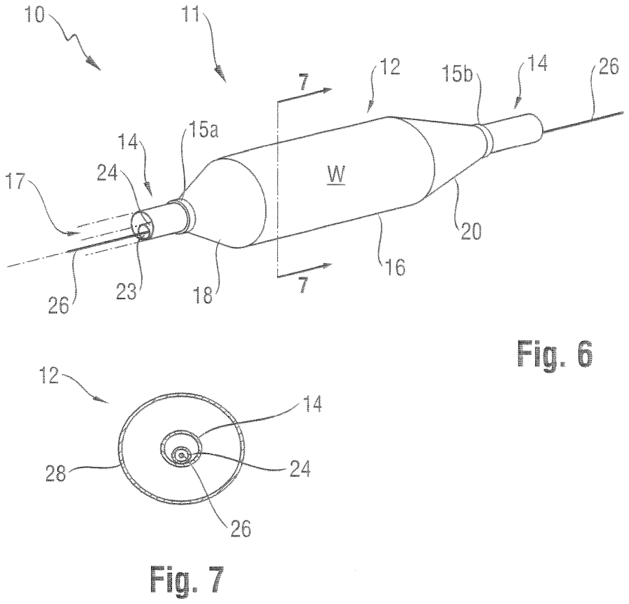

Provided is a catheter 10 having a distal portion 11 with a balloon 12 mounted on a catheter tube 14. Referring to Figures 6, 7, and 8, the balloon 12 has an intermediate section 16, or "barrel," and end sections 18, 20. In one embodiment, the end sections 18, 20 reduce in diameter to join the intermediate section 16 to the catheter tube 14 (and thus sections 18, 20 are generally termed cones or cone sections). The balloon 12 is sealed at balloon ends (proximal end 15a and distal end 15b) on the cone sections 18, 20 to allow the inflation of the balloon 12 via one or more inflation lumens 17 extending within catheter tube 14 and communicating with the interior of the balloon 12.

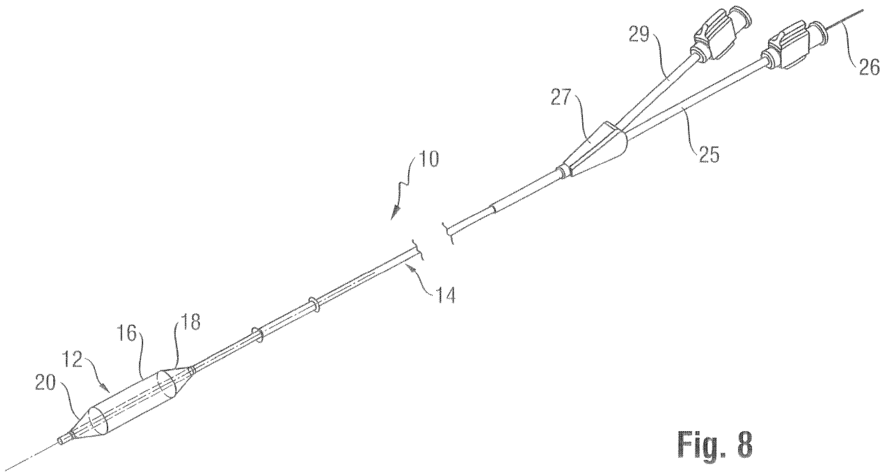

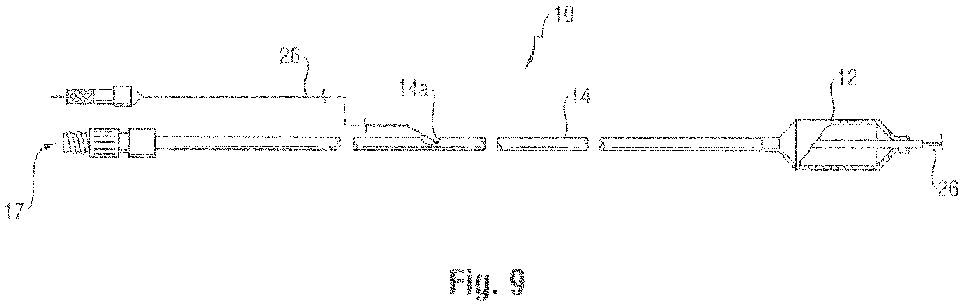

The catheter tube 14 also includes an elongated, tubular shaft 24 forming a guidewire lumen 23 that directs the guidewire 26 through the catheter 10, and along the distal end of which the balloon 12 may be located. As illustrated in Figure 8, this guidewire 26 may extend through the proximal end of the catheter 10 and a first port 25 of a connector 27 into the lumen 23 to achieve an "over the wire" (OTW) arrangement, but could also be provided in a "rapid exchange." (RX) configuration, in which the guidewire 26 exits a lateral opening 14a closer to the distal end (see Figure 9) or else is fed through a passage at the tip distally of the balloon 12 ("short" RX; not shown) . A second port 29 may also be associated with catheter 10, such as by way of connector 27, for introducing a fluid (e.g., saline, a contrast agent, or both) into the interior compartment of the balloon 12 via the inflation lumen 17.

Balloon 12 may include a single or multi-layered balloon wall 28 forming the interior for receiving the inflation fluid. The balloon 12 may be a non-compliant balloon having a balloon wall 28 that maintains its size and shape in one or more directions when the balloon is inflated. Examples of non-compliant balloons may be found in U.S. Pat. No. 6,746,425 and Publication Nos. US 2006/0085022, US 2006/0085023 and US 2006/0085024. The balloon 12 in such case also has a pre-determined surface area that remains constant during and after inflation, also has a pre-determined length and predetermined diameter that each, or together, remain constant during and after inflation. However, the balloon 12 could be semi-compliant or compliant instead, depending on the particular use.

In order to provide an enhanced locatability during an interventional procedure, the balloon 12 may have a radiopaque quality. In one embodiment, this radiopaque quality is provided in a manner that allows for a clinician to differentiate, with relative ease and high precision, one portion of the balloon 12 from another (such as the barrel section 16 including the working surface W from the cone sections 18, 20). This helps the clinician ensure the accurate positioning of the balloon 12 and, in particular, the working surface W, at a specified treatment location, which may be especially important in the delivery of drugs via the balloon surface, as outlined in more detail in the following description.

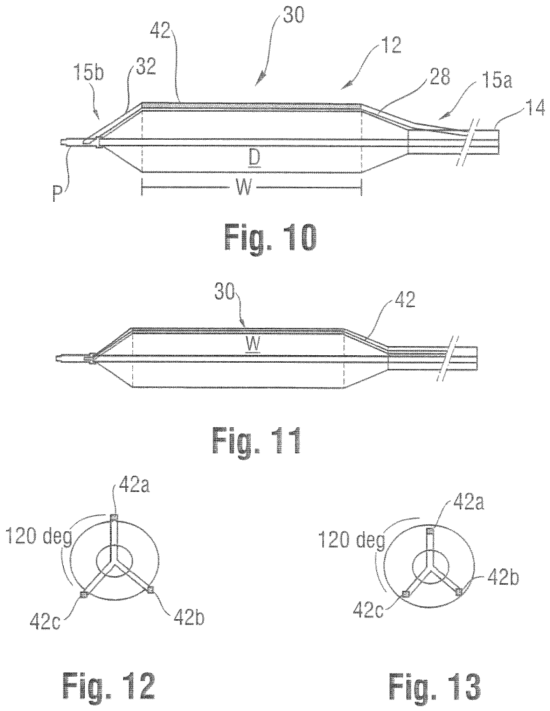

The identification of a desired portion of a balloon 12 may be achieved by providing an identifier 30 created by a wire 42 that is at least partially radiopaque, as shown in Figure 10 (radiopacity of identifier 30 is shown darkened for purposes of illustration). The wire 42 may extend along the balloon wall 28. More than one wire 42 may be provided, such as for example three wires 42a, 42b, 42c, but this is not a limit (e.g., possibly four, five, or six or more; not shown). The plural wires, such as wires 42a, 42b, 42c, may be positioned equidistantly apart in the circumferential direction (e.g., two an 180 degrees, three at 120 degrees, four at 90 degrees), with one or more of the wires being along the exterior surface (Figure 12), within the interior (Figure 13), or a mixture of both arrangements (not shown). In any case, the proximal and distal ends of the wire 42 may be captured in place by being included in or connected to the bond used to seal the balloon 12 at the distal and proximal ends 15a, 15b (such as to the tube 14 and tip P, respectively).

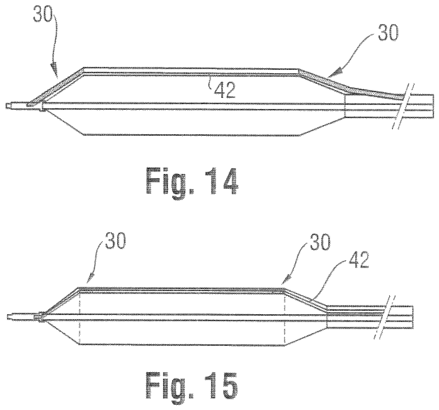

Different portions of the wire 42 may serve to provide the radiopacity, such as for identifying the working surface W in the balloon 12. For example, as shown in Figures 10 or 11, the radiopacity may be provided along the entire portion of the wire 42 corresponding to the working surface W to create a corresponding identifier 30. Alternatively, the radiopacity may be provided along the portions of the wire 42 other than along the working surface W, as shown by the identifiers 30 in Figure 14. Still another option is to provide the radiopaque portions of the wire 42 at the ends of the working surface W only, such as in the form of segments or bands, with an intermediate portion of the wire 42 extending along the working surface including no added radiopaque material, as shown in Figure 15.

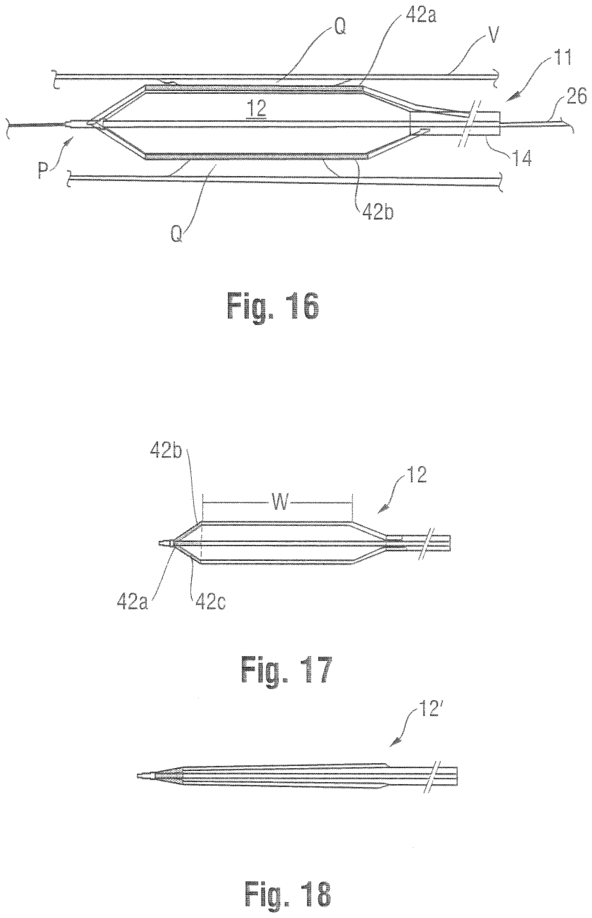

In addition to being radiopaque, the wire 42 may be used to provide a focused force for contacting a structure in a vessel during use. Thus, for example, an external wire 42 in the Figure 10 embodiment may be used to contact a plaque Q in a vessel V when the balloon 12 is fully or partially inflated. The use of two such wires 42a, 42b is shown in Figure 16. In such uses, the wire(s) 42 may be made of metal or a like rigid material to provide the desired focused force for contacting the plaque Q, but the wire(s) could also be made of polymer materials. When using the embodiment in which the entire portion of the wire 42 corresponds to the working surface W, it should also be appreciated that the fluorescence of this portion of the wire(s) 42 contacting the structure in the vessel V allows for the clinician to ensure that the contact is being made in the desired fashion. Additional details may be found in International Application WO 07/132447, as well as by referencing the VASCUTRAK product of Bard Peripheral Vascular, Inc., of Tempe, Arizona.

The presence of multiple wires, each having a radiographic quality, may help to distinguish between the flattened or pancaked condition and the inflated condition. For instance, the presence of three wires 42a, 42b, 42c having a radiopaque portion extending along the cone sections 18, 20, as shown in Figure 17, would cause the balloon 12 when deflated to appear remarkably different than in the inflated condition, as can be seen in balloon 12' in Figure 28.

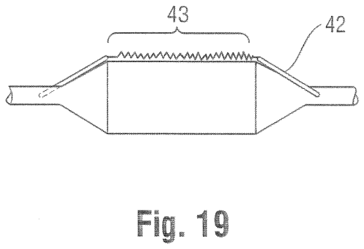

In one particular embodiment, the wire 42 or wires could be fabricated of special material s or combinations of materials. For instance, the wire 42 could be constructed having a segment that is fully or partially radiopaque, which segment 43 is compliant or semi-compliant (e.g., an elastic material or spring, which may be formed of a radiopaque material (polymer or metal); see schematic of Figure 19). The material may be chosen such that a stiffness of the segment 43 matches the compliance of the balloon 12, which is typically formed of a polymer, such as Nylon 12. Consequently, any longitudinal or radial elongation of the balloon working surface W under pressure would be matched by the radiopaque segment, with the remaining portions of the wire 42 being non-compliant and attached in place as previously described. Alternatively, the intermediate segment 43 of the wire 42 could be radiopaque (polymer or metal), but non-complaint, and the other portions made compliant (polymer or metal), to provide the desired match with the working surface W. Still another option is to make the intermediate portion compliant, and the ends of the wire radiopaque and non-compliant. In the case where different materials are used to form the wire 42 along its length, such as a polymer segment with metal ends, the bonding may be accomplished by shrink-fitting a tubular end of one segment over the end of an adjacent segment to create a substantially continuous wire 42.

One or more of the wires 42 may comprise a shape-memory material, such as NITINOL. Such materials may be set using temperature to achieve certain shapes. Thus, an external wire 42 could be arranged to lay in a nominal condition prior to full inflation (e.g., Figure 16), and then temperature activated (such as by the contrast fluid) to match the shape of the balloon 12 when fully inflated (see Figure 11). When deflated, the temperature of the surrounding blood and tissue could be used to re-activate the original memory and straighten the wire 42.

The wire 42 or wires may also be used in connection with other radiopaque identifiers, such as patterns, bands, strips or the like. Details may be found in co-pending applications filed on the same date as this application, entitled **"MEDICAL BALLOON WITH RADIOPAQUE IDENTIFIER FOR PRECISELY IDENTIFYING THE WORKING SURFACE,**" for inventors Scan Wall, Pat Byrne, Robert Righi, Angela Crall, Paul Wales, and Allan Ronan, and **"MEDICAL BALLOON WITH RADIOPAQUE END PORTION FOR PRECISELY IDENTIFYING A WORKING SURFACE LOCATION,**" for inventors Sean Wall, Scott Randall, Robert Righi, Angela Crall.

Balloons 12 that incorporate coatings comprising drugs to be applied to the vasculature may also benefit from the above-referenced embodiments. For example, as shown in Figure 10, a balloon 12 including a defined working surface W, such as by providing radiopaque markings 30 at the transitions between the barrel portion 16 and cone portions 18, 20, may include portion coated with such a drug D, such as one designed for achieving a desired therapeutic effect when applied to the interior of the vessel. The drug D may be applied to the inflated balloon as part of the manufacturing process, and prior to folding for insertion in the vasculature. The clinician may thus with the benefit of a fluoroscope determine the precise positioning of the working surface W prior to inflating the balloon 12 in the vasculature to deliver the drug D to the desired location and provide the desired treatment regimen.

Examples of suitable radiopaque materials include, but are not limited to, barium, bismuth, tungsten, iridium, iodine, gold, iron, or platinum. The amounts used may vary depending on the desired coverage and the desired degree of radiopacity.

## Claims

1. A balloon catheter (10) for use in connection with a guidewire, comprising:
an elongated, tubular shaft (24) extending in a longitudinal direction, said shaft having a proximal end and a distal end;
an inflatable balloon (12) supported along the distal end of the shaft (24), the balloon when inflated including first and second spaced ends and a working surface (W) between the ends; and
a plurality of wires extending generally in the longitudinal direction, at least one of the plurality of wires (42) including at least a radiopaque portion for identifying the location of working surface (W) of the balloon,
wherein the at least one wire is at least partially elastic.

2. The catheter of claim 1, wherein said at least one wire comprises a material having a shape memory for adjusting between a first state and a second state.

3. The catheter of any of the foregoing claims, wherein the radiopaque portion is elongated.

4. The catheter of any of the foregoing claims, wherein
at least one of the plurality of wires at least partially comprises a polymer.

5. The catheter of any of the foregoing claims, wherein radiopaque portions, preferably in the form of segments or bands, of the at least one wire (42) are provided at the ends of the working surface (W), optionally with an intermediate portion of the at least one wire (42) extending along the working surface (W) free from added radiopaque material.

6. The catheter of any of the foregoing claims, wherein at least one of the plurality of wires extends along an outer surface of the balloon.

7. The catheter of any of the foregoing claims, wherein at least one of the plurality of wires extends along an inner surface of the balloon.

8. The catheter of any of the foregoing claims, wherein the at least one wire extends from the first end to the second end of the balloon.

9. The catheter of any of the foregoing claims, wherein the radiopaque portion of the at least one wire extends along a portion of the balloon corresponding to the working surface.

10. The catheter of any of the foregoing claims, wherein the radiopaque portion of at least one wire extends along other than along the portion of the balloon corresponding to the working surface.

11. The catheter of any of the foregoing claims , wherein the plurality of wires are spaced substantially equidistantly around a circumference of the balloon.

12. The catheter of any of the foregoing claims, wherein at least one wire includes a compliant or semi-compliant portion.

13. The catheter of any of the foregoing claims, wherein the catheter includes a drug provided on the balloon.

14. The catheter of any of the foregoing claims, wherein at least one end of the at least partially radiopaque wire is attached to a bond connecting the balloon to the shaft.

## Patentansprüche

1. Ballonkatheter (10) zur Verwendung in Verbindung mit einem Führungsdraht, umfassend:
einen länglichen rohrförmigen Schaft (24), der sich in einer Längsrichtung erstreckt, wobei der Schaft ein proximales Ende und ein distales Ende aufweist;
einen aufblasbaren Ballon (12), der entlang des distalen Endes des Schafts (24) gestützt wird, wobei der Ballon, wenn er aufgeblasen ist, erste und zweite beabstandete Enden und eine Arbeitsfläche (W) zwischen den Enden beinhaltet; und
eine Vielzahl von Drähten, die sich im Allgemeinen in der Längsrichtung erstrecken, wobei mindestens einer der Vielzahl von Drähten (42) mindestens einen strahlenundurchlässigen Abschnitt zum Identifizieren der Stelle der Arbeitsfläche (W) des Ballons beinhaltet,
wobei der mindestens eine Draht mindestens teilweise elastisch ist.

2. Katheter nach Anspruch 1, wobei der mindestens eine Draht ein Material mit einem Formgedächtnis zum Einstellen zwischen einem ersten Zustand und einem zweiten Zustand umfasst.

3. Katheter nach einem der vorstehenden Ansprüche, wobei der strahlenundurchlässige Abschnitt länglich ist.

4. Katheter nach einem der vorstehenden Ansprüche, wobei mindestens einer der Vielzahl von Drähten mindestens teilweise ein Polymer umfasst.

5. Katheter nach einem der vorstehenden Ansprüche, wobei strahlenundurchlässige Abschnitte, vorzugsweise in der Form von Segmenten oder Bändern, des mindestens einen Drahts (42) an den Enden der Arbeitsfläche (W) bereitgestellt sind, wobei sich gegebenenfalls ein Zwischenabschnitt des mindestens einen Drahts (42) entlang der Arbeitsfläche (W) frei von zugesetztem strahlenundurchlässigem Material erstreckt.

6. Katheter nach einem der vorstehenden Ansprüche, wobei mindestens einer der Vielzahl von Drähten sich entlang einer äußeren Oberfläche des Ballons erstreckt.

7. Katheter nach einem der vorstehenden Ansprüche, wobei mindestens einer der Vielzahl von Drähten sich entlang einer inneren Oberfläche des Ballons erstreckt.

8. Katheter nach einem der vorstehenden Ansprüche, wobei der mindestens eine Draht sich von dem ersten Ende zu dem zweiten Ende des Ballons erstreckt.

9. Katheter nach einem der vorstehenden Ansprüche, wobei der strahlenundurchlässige Abschnitt des mindestens einen Drahts sich entlang eines Abschnitts des Ballons, der der Arbeitsfläche entspricht, erstreckt.

10. Katheter nach einem der vorstehenden Ansprüche, wobei der strahlenundurchlässige Abschnitt von mindestens einem Draht sich anders als entlang des Abschnitts des Ballons, der der Arbeitsfläche entspricht, erstreckt.

11. Katheter nach einem der vorstehenden Ansprüche, wobei die Vielzahl von Drähten um einen Umfang des Ballons im Wesentlichen gleich beabstandet sind.

12. Katheter nach einem der vorstehenden Ansprüche, wobei der mindestens eine Draht einen nachgebenden oder halb nachgebenden Abschnitt beinhaltet.

13. Katheter nach einem der vorstehenden Ansprüche, wobei der Katheter ein Arzneimittel beinhaltet, das auf dem Ballon bereitgestellt ist.

14. Katheter nach einem der vorstehenden Ansprüche, wobei mindestens ein Ende des mindestens teilweise strahlenundurchlässigen Drahts an einer den Ballon mit dem Schaft verbindenden Verbindung befestigt ist.

## Revendications

1. Cathéter à ballonnet (10) destiné à être utilisé avec un fil-guide, comprenant :
une tige tubulaire allongée (24) s'étendant dans une direction longitudinale, ladite tige présentant une extrémité proximale et une extrémité distale ;
un ballonnet gonflable (12) supporté le long de l'extrémité distale de la tige (24), le ballonnet lorsqu'il est gonflé incluant des première et seconde extrémités espacées et une surface de travail (W) entre les extrémités ; et
une pluralité de fils s'étendant généralement dans la direction longitudinale, au moins un fil de la pluralité de fils (42) incluant au moins une partie radio-opaque pour identifier l'emplacement d'une surface de travail (W) du ballonnet,
dans lequel le au moins un fil est au partiellement élastique.

2. Cathéter selon la revendication 1, dans lequel ledit au moins un fil comprend un matériau présentant une mémoire de forme pour un ajustement entre un premier état et un second état.

3. Cathéter selon l'une quelconque des revendications précédentes, dans lequel la partie radio-opaque est allongée.

4. Cathéter selon l'une quelconque des revendications précédentes, dans lequel au moins un fil de la pluralité de fils comprend au moins partiellement un polymère.

5. Cathéter selon l'une quelconque des revendications précédentes, dans lequel des parties radio-opaques, de préférence sous la forme de segments ou de bandes, du au moins un fil (42) sont disposées au niveau des extrémités de la surface de travail (W), facultativement avec une partie intermédiaire du au moins un fil (42) s'étendant le long de la surface de travail (W) dépourvue de matériau radio-opaque ajouté.

6. Cathéter selon l'une quelconque des revendications précédentes, dans lequel au moins un fil de la pluralité de fils s'étend le long d'une surface externe du ballonnet.

7. Cathéter selon l'une quelconque des revendications précédentes, dans lequel au moins un fil de la pluralité de fils s'étend le long d'une surface interne du ballonnet.

8. Cathéter selon l'une quelconque des revendications précédentes, dans lequel le au moins un fil s'étend depuis la première extrémité jusqu'à la seconde extrémité du ballonnet.

9. Cathéter selon l'une quelconque des revendications précédentes, dans lequel la partie radio-opaque du au moins un fil s'étend le long d'une partie du ballonnet correspondant à la surface de travail.

10. Cathéter selon l'une quelconque des revendications précédentes, dans lequel la partie radio-opaque du au moins un fil s'étend le long d'une autre partie que le long de la partie du ballonnet correspondant à la surface de travail.

11. Cathéter selon l'une quelconque des revendications précédentes, dans lequel la pluralité de fils sont espacés sensiblement de manière équidistante autour d'une circonférence du ballonnet.

12. Cathéter selon l'une quelconque des revendications précédentes, dans lequel le au moins un fil inclut une partie souple ou semi souple.

13. Cathéter selon l'une quelconque des revendications précédentes, dans lequel le cathéter inclut un médicament disposé sur le ballonnet.

14. Cathéter selon l'une quelconque des revendications précédentes, dans lequel au moins une extrémité du fil au moins partiellement radio-opaque est fixée à une liaison reliant le ballonnet à la tige.
